# EUROPEAN PATENT SPECIFICATION

(11) **EP 1 651 984 B1**
(45) Date of publication and mention of the grant of the patent: **29.02.2012**
(21) Application number: 03817389.4
(22) Date of filing: 02.07.2003
(51) Int. Cl.: G01T 1/29, H01J 47/02

(54) **MULTIFUNCTIONAL DETECTOR FOR MEASURING CHARACTERISTICS OF THE BEAM OF PARTICLES OR RADIATION**
MEHRFUNKTIONSDETEKTOR ZUR MESSUNG VON EIGENSCHAFTEN DES STRAHLS VON PARTIKELN ODER STRAHLUNG
DETECTEUR MULTIFONCTIONNEL DESTINE A MESURER DES CARACTERISTIQUES D'UN FAISCEAU DE PARTICULES OU DE RAYONNEMENT

(43) Date of publication of application: 03.05.2006
(73) Proprietor: European Organisation for Nuclear Research CERN, 1211 Geneva 23 (CH)
(72) Inventor: HORI, Masaki, CH-1211 Geneva 23 (CH); MITANI, Minoru, Minotos Corporation, Kunitachi-shi, Tokyo 186-0011 (JP); HAYANO, Ryugo, University of Tokyo, Tokyo 113-0033 (JP)
(74) Representative: Bittner, Thomas L.
(86) International application number: PCT/EP2003/007064
(87) International publication number: WO 2005/006017

(56) References cited:
- EP-A- 0 450 571
- US-A- 4 345 155
- US-A- 5 731 584
- US-A- 5 892 227
- US-A1- 2002 030 289

## Description

The invention relates to a multifunctional position-sensitive detector for measuring characteristics of a beam of particles or radiation, particularly to a position-sensitive detector for measuring images of spatial profile, intensity, dose, or timing profile of a beam of charged particle or of ionizing radiation and to a method to produce such a position sensitive detector. It further relates to patterned electrodes to be used in such a multifunctional detector and a method to produce them.

### Background of the invention

Position-sensitive detectors for measuring characteristics and parameters, in particular profiles of radiation and particle beams are well known in the prior art. Among the detectors used so far are ionization chambers, micro-strip detectors, gas electron multipliers, secondary electron emission chambers, position-sensitive Faraday cups, multi-wire proportional chambers, and parallel plate avalanche chambers. The above mentioned detector devices are each optimized for measuring specific types of radiation or particles with a certain range of energies and intensities. For example, ionization chambers have relatively low sensitivity and are therefore limited to detecting beams with intensities greater than a few pico-amperes. Multi-wire proportional chambers, parallel plate avalanche chambers, and gas electron multipliers have a higher sensitivity, which enables them to detect single particles; however, they can not be used to measure high-intensity beams which induce electric space-charge effects. All four of these gas detectors are typically limited to measuring beams with energies of at least a few hundred kilo electron-volts, which can penetrate the windows of the detector and traverse the gas inside it. Faraday cups, on the other hand, can be used in vacuum and measure beams of nearly zero energy. Secondary electron emission chambers are operated in vacuum to detect higher-energy beams.

An important aspect of these detectors is their transparency to radiation and particle beams. During radiation-therapy involving the irradiation of cancer tumors, a position-sensitive detector is often placed in the beam directly in front of the patient, so that spatial profile, intensity or dosage, and time profile of the beam can be monitored throughout the treatment. It is essential that the detector itself does not scatter, degrade, or attenuate the beam, in order to ensure that only the tumor is irradiated accurately, and the healthy cells around are left undamaged. The 'amount of material in the detector must therefore be minimized, so that the beams travelling through it are not disturbed by its presence.

Ionization chambers have been frequently used in past radiation-therapy applications. These detectors, however, require a constant flow of gas such as argon or carbon dioxide for operation, which are often supplied by industrial gas storage cylinders weighing hundreds of kilograms and containing pressurized gases between 100 and 200 Bars. It has been often found cumbersome and expensive to ensure the safe and proper handling of these gases in medical facilities, and to exchange the storage cylinders when the gas has been fully depleted.

The majority of the above detectors are, moreover, constructed using materials and fabrication techniques that introduce a large outgasing contamination, which excludes their use in ultraclean or ultra-high vacuum environments such as semiconductor manufacturing facilities. Most of the detectors cannot be used in some or all of the following extreme and hostile environments having high pressures, high magnetic fields, high temperatures, or cryogenic temperatures.

The position-sensitive detectors in prior art are often expensive and specialized due to their complex construction. Electrodes of large size and/or resolution are produced by expensive and time-consuming processes such as photolithography, patterned evaporation of metals in a vacuum chamber, or the soldering of wires by hand. A detector with a more simple construction, which can be cheaply mass-produced is clearly desired.

When characterizing particle beams or electromagnetic radiation of various types, energies, and intensities, it is desirable to modify the shapes, thickness, and resolutions of the electrodes comprising the position-sensitive detector. For example, foil electrodes are often preferred in medical application where cancer patients are irradiated with particle beams of relatively high energies and intensities. The planar geometry guarantees that the particle beams will be decelerated or scattered by an uniform amount, irrespective of their point of passage on the electrode surface, and so would emerge with a smooth spatial profile and uniform energy. In other applications, however, electrodes made of wires may be preferred. When irradiating small tumors, a detector with electrodes of high spatial resolution and small active area may be required, whereas when treating large tumors, lower spatial resolutions and larger activity areas may be needed.

The document EP 0 450 571 A2 discloses an X-ray detector comprising a low pressure gas filled chamber including a gas entry conduit, an X-ray photon entrance window and a gas exit conduit. X-ray photons passing through the photon entrance window impinge on a photocathode. The impingement of the X-ray photons on the photocathode causes the release of electrons from the photocathode at the location of the impingement. The released electrons are amplified in a pre-amplification stage to produce an initial avalanche of electrons. The electron avalanche is transferred via a transfer gap to an amplification stage, which produces a second avalanche. The electrons in the second avalanche are transferred to a second transfer stage and are collected by an array of pad electrodes, which are connected to readout electronics.

### Invention

It is an object of the present invention to provide an improved position-sensitive detector that is more versatile and general-purpose compared to detectors of the prior art and avoiding their deficiencies. It is further an object of the invention to provide a method for manufacturing said improved, more versatile and general purpose position-sensitive detector.

According to a first aspect of the invention a position-sensitive detector for measuring characteristics of a beam of particles or radiation is provided. The position-sensitive detector comprises: a chamber; and a plurality of electrodes arranged in a parallel configuration inside said chamber, said plurality of electrodes comprises a plurality of patterned electrodes, wherein each of said plurality of patterned electrodes comprises a pattern of segments electrically insulated with respect to each other, and each of said segments being separately electronically connectable; characterized in that said chamber comprises multi-connector means for individually detachably connecting each of said plurality of patterned electrodes electrically to said multi-connector means inside said chamber.

The disclosed position-sensitive detector can be operated in a variety of detector modes. Thereby the detector can measure parameters and characteristics of particle beams, such as electrons, positrons, protons, antiprotons, ions, muons, and pions. It can detect particle beams with intensities ranging from single particles up to 10¹³ particles in pulses as short as 100 nanoseconds and with energies between about 1 electron-volt and about 100 giga-electron-volts. The detector is further capable of measuring beam characteristics of electromagnetic radiation beams, such as gamma-rays, X-rays, and ultraviolet light. Therefore, the detector can be applied in numerous applications in radiation-therapy, ion and electron lithography, second electron emission microscopy, ultraviolet and gamma-ray cameras, and diagnostics of particle accelerators.

In contrast to position-sensitive detectors of the prior art that comprise electrodes which are permanently bonded, glued, soldered, or vacuum-sealed into a chamber, making it difficult or impossible for the user in the field to rearrange their configuration, the electrodes in the disclosed detector can easily be exchanged by the user in the field. The necessity to provide separate detectors for each electrode configuration is omitted. In this multi-purpose detector electrodes can easily be exchanged as desired so that the detector performance can be optimized on the particle beams or electromagnetic radiation being measured.

According to a second aspect of the invention a method for manufacturing a position-sensitive detector comprising a plurality of electrodes is provided. The method comprises the steps of: supplying a chamber; producing a plurality of patterned electrodes being comprised in said plurality of electrodes, wherein the process of producing said plurality of patterned electrodes comprises the steps of: supplying an insulating substrate frame; depositing a metallic pattern on said insulating substrate frame; and attaching segments to said metallic pattern to define a pattern of said patterned electrode; and arranging said plurality of electrodes in a parallel configuration inside said chamber; characterized by providing multi-connector means and individually detachably connecting each of said plurality of patterned electrodes electrically to said multi-connector means.

According to a third aspect of the invention a method for producing a patterned electrode is provided comprising the steps of: supplying an insulating substrate frame; depositing a metallic pattern on said insulating substrate frame; attaching segments to said metallic pattern to define a pattern of said patterned electrode; and configuring said patterned electrode so that said patterned electrode can be individually detachably electrically connected to multi-connector means.

By these methods a detector with patterned electrodes and the patterned electrodes itself are provided, that are very robust, and can therefore be used in a variety of extreme and hostile environments, such as high pressures up to 10 Bars, magnetic fields up to 5 Tesla, and temperatures between - 273 and 200 degrees Celsius. The methods are very clean, such that the resulting detector and/or patterned electrode are not outgasing in an ultrahigh vacuum. The methods are very suitable for producing position-sensitive detectors and/or patterned electrodes in a very cost-effective way.

According to a fourth aspect of the invention a patterned electrode is provided, which is configured to be individually detachably electrically connected to multi-connector means. The patterned electrode comprises: an insulating frame; a metallic pattern deposited on said frame; and segments connected to said metallic pattern.

This type of patterned electrode is particularly suited to be used in a versatile position-sensitive detector.

A preferred embodiment of the position-sensitive detector is characterized in that said multi-connector means are vacuum feedthroughs.

This embodiment is advantageous since the length of the line the electrical signals being detected have to travel is minimized.

A further preferred embodiment of the invention is characterized in that said plurality of patterned electrodes is suspended by said multi-connector means.

This embodiment comprises an easy mechanical construction. The amount of structural features inside the vacuum chamber is reduced, which improves the vacuum performance of the detector system by reducing the total amount of surface being present in the chamber.

It is advantageous, that the patterned electrodes, for example micro-wire and/or foil electrodes, can be easily exchanged by the user in the field thus allowing the position-sensitive detector to be reconfigured.

A preferred embodiment of the position-sensitive detector is characterized in that said plurality of electrodes comprises foil electrodes and/or micro-wire electrodes only.

The advantage of this embodiment is that said plurality of electrodes introduces very little degradation, attenuation, or scattering to the radiation or particle beams it measures. This is essential in radiation-therapy applications described above. The position-sensitive detector can thus be configured as: i) said chamber containing at least one micro-wire electrode and at least one foil electrode, ii) said chamber containing only foil electrodes, or iii) said chamber containing only micro-wire electrodes. A configuration of said electrodes can thus be optimized to measure said beam of particles or radiation of different types, energies, and intensities.

A further preferred embodiment of the invention is characterized in that said foil electrodes are all patterned foil electrodes.

In this type of position-sensitive detectors each of the plurality of electrodes comprised can also be used for position-sensitive measurements. This way the total number of electrodes needed for a certain variety of different modes to operate said detector in can be minimized.

Another more preferred embodiment of the invention is characterized in that each of said segments of said plurality of patterned electrodes is connected to a separate readout circuit.

By reading out each of said segments of said plurality of patterned electrodes with a separate readout circuit the maximum possible information supplied by said patterned electrodes can be extracted for each measurement. Thereby a high precision measurement with respect to the different characteristics measured by said detector according to the invention can be reached, that is superior compared to measurements carried out by detectors according to the prior art.

Another advantageous embodiment of the invention is characterized by means for evaluating electronic signals from said readout circuits connected to said means, which electronically deduced at least one parameter characterizing said beam.

This embodiment supplies at least one parameter characterizing the beam to be measured. Since the deduction is done electronically, said parameter(s) will be available almost simultaneously with the measurement carried out. Such a detector is therefore very suited for applications, in which the characteristic or parameter of the beam has to be monitored online, for example, in radiation-therapy, or for monitoring particle beams in accelerators. Such a detector can also be used as an input to a control-system using a feedback loop to influence the beam itself.

A further preferred embodiment of the invention is characterized in that said chamber comprises a gas inlet for inserting gas into said chamber and a gas outlet for evacuating said chamber.

By comprising a gas inlet and a gas outlet in the detector chamber the detector can be operated in "gas filled"-modes and in "evacuated"-modes of operation. This further increases the flexibility with respect to the different operational modes the detector can be used in. Another preferred embodiment of the invention is characterized in that said foil electrode separates said chamber into a portion and another portion, being substantially hermetically sealed with respect to fluid exchange between each other.

This embodiment of the invention provides two detectors that can be used separately. The patterned foil electrode acts on one hand as a window that physically inhibits gas molecules and atoms to be exchanged between the two portions of said chamber. On the other hand it measures the spatial profile, intensity, and time structure of the radiation or particle beams passing between the two portions of said chamber at the same time.

Another more preferred embodiment of the invention is characterized by a voltage supply to bias at least one of said plurality of electrodes.

By biasing at least one of said plurality of electrodes the detector is capable to detect particle and/or radiation beams. By biasing the different electrodes comprised by the detector a large variety of measurement modes can be realized.

A preferred embodiment of the methods according to the second or third aspect of the invention is characterized in that said step of attaching said segments to said metallic pattern comprises the steps of: aligning said segments on said metallic pattern; applying a conductive paste, in particular composed of silver-carbon, on top of said metallic pattern and said segments at an interface between them; and electrically connecting said segments and said metallic pattern by said applied conductive paste.

These steps for attaching the segments to the metallic pattern ensure a reliable electric connection between the segments and said metallic pattern. At the same time the process is very clean with respect to outgasing, when the detector chamber is evacuated to ultrahigh vacuum, or when the patterned electrode is used in ultrahigh vacuum conditions.

Another preferred embodiment of the methods according to the second or third aspect of the invention comprises the steps of supplying a plastic foil being transparent to laser-light; applying a metal layer to said foil; and scanning laser-light across said metal layer to vaporize parts of said metal layer thereby patterning said metal layer into said segments without destroying said plastic foil.

By producing a patterned foil electrode using the process just mentioned metal layer segments of arbitrary shape can be manufactured to high precession. At the same time the electrode resulting from this process contains only a minimal amount of material necessary to function as segmented electrode. This way a degradation or attenuation of a beam of particle or a beam of radiation is minimized.

One preferred embodiment of the invention according to the fourth aspect of the invention is characterized in that an integrated circuit comprising a readout circuit for each of said segments is connected directly to the metallic pattern on said frame.

This way noise and interference introduced on transmission lines connecting the segmented electrode with a readout circuit can be eliminated or reduced drastically. This way the sensitivity of the detector using this kind of patterned electrode is increased.

Other features and advantages of the present invention will be come apparent to a person with ordinary skill in the art in view of the following drawings and detailed description. It is intended that all such additional features and advantages be included herein within the scope of the present invention.

### Drawings

Exemplary embodiments of the invention illustrated in the drawings are explained in more detail in the following description.

In the figures:
- Figure 1a: illustrates a schematic drawing of a preferred embodiment;
- Figure 1b: illustrates a schematic detail of the embodiment displayed in figure 1a, showing the connection of patterned electrodes to multi-connector means;
- Figure 2a: shows a front view of another preferred embodiment, in which a gas may be introduced into the chamber to operate as ionization chamber, parallel plate avalanche chamber, or multi-wire proportional chamber;
- Figure 2b: displays a cross-section along the line A-A of figure 2a;
- Figure 3-: illustrates a cross-section of a preferred embodiment of the position-sensitive detector, in which the chamber is evacuated to vacuum allowing it to operate as a Faraday cup or as secondary electron emission chamber;
- Figure 4a: shows a detailed front view of the patterned foil electrode depicted in figure 1a;
- Figure 4b: shows a detailed cross-section of the patterned foil electrode depicted in figure 4a;
- Figure 4c: is a microscopic view of the patterned foil electrode depicted in figure 4a;
- Figure 4d: is a perspective view representing the manufacturing of the patterned foil electrode depicted in figure 4a, in which a laser beam selectively vaporizes the metal layer on the plastic foil;
- Figure 5a: illustrates a front view of a micro-wire electrode depicted in figure 1a;
- Figure 5b: shows a detailed cross-section of the micro-wire electrode depicted in figure 5a;
- Figure 6: is a schematic diagram representing the electronic components comprising a readout circuit and means for evaluating the signal from said read out circuit;
- Figure 7: illustrates a diagram representing a preferred embodiment of the position-sensitive radiation detector, wherein electronic signals induced on the electrodes are carried by signal transmission lines and vacuum feedthroughs to remote readout circuits;
- Figure 8: is a diagram representing a preferred embodiment of the position-sensitive detector, in which the readout circuits are miniaturized and encapsulated into integrated circuits, which are then mounted directly on or near the foil electrodes or micro-wire electrodes;
- Figure 9: illustrates a cross-section of a preferred embodiment of the position-sensitive detector, wherein a foil electrodes function as a barrier separating a chamber into two portions, which can be filled with gas separately.

### Detailed description of the invention

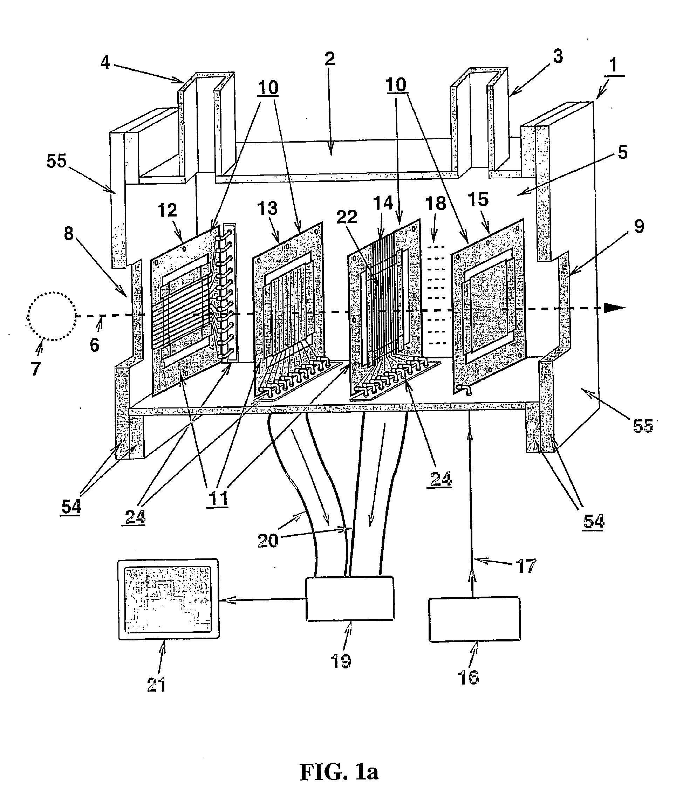

Figure 1a shows a schematic drawing of a preferred embodiment. A position-sensitive detector 1 comprises a chamber 2. The chamber 2 comprises a gas inlet 3 and a gas outlet 4. The gas inlet 3 can be used to fill the chamber 2 with a gas 5. The gas outlet 4 in turn can be used to evacuate the chamber 2. It can also be used in a connection with the gas inlet 3 to establish a constant flow of the gas 5 within the chamber 2.

A beam 6 emitted from a source 7 enters into the chamber 2 through an entrance opening 8 traverses the chamber 2 and exits the chamber 2 via an exit opening 9. The beam 6 may be a particle beam of any kind comprising, for example, electrons, positrons, muons, protons, ions, or a radiation beam, for example, a gamma-ray, X-ray, or ultraviolet radiation.

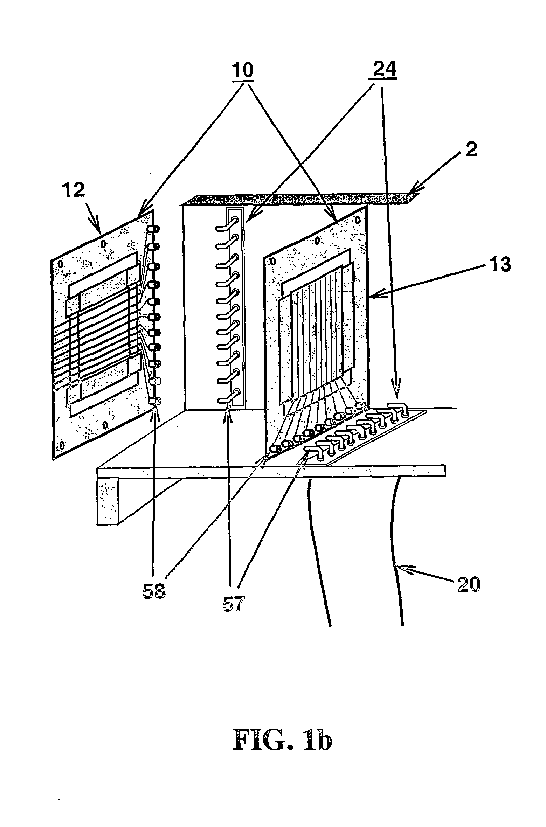

In traversing the chamber 2 the beam 6 passes through a plurality of electrodes 10. This plurality of electrodes 10 can comprise a plurality of patterned electrodes 11. This plurality of patterned electrodes 11 comprises a micro-wire electrode 12 and a patterned foil electrode 13. The plurality of patterned electrodes 11 can comprise further patterned foil electrodes or micro-wire electrodes. In the embodiment of figure 1 the plurality of patterned electrodes 11 comprises further one other micro-wire electrode 14. The plurality of electrodes 10 comprises in this embodiment also further an unpatterned foil electrode 15. It is preferred that the position-sensitive detector 1 only comprises foil electrodes, patterned 13 or unpatterned 15, and micro-wire electrodes 12, 14. This insures that only very little material has to be passed through by the beam 6, which in turn minimizes any degradation or attenuation of said beam 6.

Vacuum manifolds 55 are attached to the vacuum chamber 2 by vacuum flanges 54. Normally the vacuum flanges 54 hermetically seal the chamber 2, thus inhibiting the gas 5 from escaping to the outside of the chamber 2. Moreover, the user in the field can easily detach at least one of the vacuum manifolds 55 from the vacuum chamber 2 along the vacuum flanges 54, and thus access the plurality of electrodes 10 inside the chamber 2. Each of the plurality of electrodes 10 is mechanically held into position by multi-connector means 24 which in this embodiment are configured as multi-pin connectors. This way each of the plurality of electrodes 10 can easily be disconnected individually. Thus any one of the plurality of electrodes 10 can be removed from the chamber 2 quickly and be replaced by a different one of a different type, thickness, or resolution, as needed. After the configuration of the plurality of electrodes 10 has been changed, at least one of the vacuum manifolds 55 is again connected to the vacuum chamber 2 and hermetically sealed along the vacuum flanges 54. In this way the position-sensitive detector 1 can be reconfigured to be comprised of: i) only foiled electrodes 13, 15, ii) only micro-wire electrodes 14, or iii) at least the foil electrode 13 and the micro-wire electrode 14. The position-sensitive detector 1 can thus be optimized for measuring the beam 6 of charged particles and/or radiation of different types, energies, and intensities.

The plurality of electrodes is connected to a voltage supply 16 via connection lines 17. The voltage supply 16 can be used to bias one, several, or all of the electrodes of said plurality of electrodes 10 in order to create an electric field 18. In figure 1a the voltage supply causes a potential difference between the at least one micro-wire electrode 12 and the at least one patterned foil electrode 13 such that the electric field 18 is homogenous. In order to bias the plurality of electrodes 10 several voltage supplies can be used. The plurality of electrodes 10 is further connected to readout circuits 19 via transmission lines 20.

In traversing the chamber 2 the beam 6 interacts with said gas 5 and/or the plurality of electrodes, being arranged in a parallel fashion spaced apart and substantially perpendicular to the direction of the beam 6. In interacting with the gas 5 the beam 6 produces primary electrons and ions. In the electric field 18 these primary electrons and ions are accelerated and move towards the surfaces of the micro-wire electrode 12 and the patterned foil electrode 13. Thereby a current is induce in said plurality of electrodes 10. The electrical signals are transmitted via the transmission lines to the readout circuits 19, where they are processed. The readout circuits 19 are connected to a display device 21, that visualizes for example the spatial profile, the timing profile, the intensity profile, or the dose of the beam 6 detected by the position-sensitive detector 1.

The operational mode just described, in which the electric field generally does not exceed several hundred volts per millimeter is called ionization chamber mode. In this mode the detector is filled with the gas 5. The type of gases and the pressure suited to operate the detector as ionization chamber are known to persons skilled in the art.

There are several other modes, the position-sensitive detector 1 can be operated in. In a second mode, designated as multi-wire proportional chamber mode, the gas 5 is introduced via the gas inlet 3. The voltage supply 16 is adjusted to attain cylindrical electrical fields typically larger than several hundred volts per millimeter in the vicinity of micro-wires 22 of said micro-wire electrode 12 and/or said other micro-wire electrode 14. The primary electrons and the primary ions created by the beam 6 within the gas 5 are than multiplied near the surfaces of said at least one micro-wire electrode 12 and/or said other micro-wire electrode 14 via gas multiplication processes, producing large electronic signals detected by said readout circuits 19.

In an parallel plate avalanche chamber mode, the gas 5 is introduced into the chamber 2 again, and the voltage supply 16 is adjusted to obtain a parallel electric field 18 generally larger than several hundred volts per millimeter in the proximity of said at least one patterned foil electrode 13. The primary electrons and the primary ions cause electronic avalanches to occur, which are detected by the readout circuits 19.

The above mentioned modes of operation are all "gas filled"-modes, since the chamber 2 is filled with the gas 5 during operation. The following three modes are called "evacuated"-modes. While performing measurements, the chamber 2 is evacuated in these modes.

In the Faraday cup mode the chamber 2 is evacuated by the gas outlet 4, such that the gas 5 inside the chamber 2 is completely removed. In cases, where the beam 6 is composed of positive charged particles, the voltage supply 16 is adjusted to apply a small negative potential on said plurality of electrodes 10, whereas when the beam 6 is composed of negative charged particles, a positive potential is provided. The beam 6 strikes said plurality of electrodes 10 and is absorbed by them, thus producing an electric signal that is measured by the readout circuits 19.

In a secondary electron emission chamber mode the chamber 2 is evacuated again and a relatively large voltage of negative polarity is applied on said at least one micro-wire electrode 12 or/and on said at least one patterned foil electrode 13. When the beam 6 strikes the at least one micro-wire electrode 12 or/and said at least one patterned foil electrode 13 secondary electrons are emitted and the resulting electric signals are detected by the readout circuits 19.

In a last mode, designated as non-destructive low-energy particle detector mode, the foil electrodes 13, 15 are removed so that only micro-wire electrodes 3 are left in the path way of said beam 6 inside said chamber 2. For example by using micro-wire electrodes 12, 14 of very small diameters down to 1 micron and 1 mm pitch, as described below, the transmission through the detector can be has high as 99.9%, thus allowing the majority of the particle beam 6 to pass without being disturbed. A small fraction (for example 0.1%) of the beam 6 strikes the micro-wire electrodes 12, 14, thus producing signals in the same way as the Faraday cup or secondary electron emission chamber, described above, which can be detected by the readout circuits 19.

Due to the patterning of the plurality of patterned electrodes 11 it is possible to measure a position sensitive profile of the beam 6. It is therefore preferred that all or almost all of the patterns of the plurality of patterned electrodes 11, which are called segments, are connected to a separate readout circuit of said readout circuits 19.

Figure 1b shows a detail of the same embodiment as displayed in figure 1a, indicating how the plurality of electrodes 10 can be detached from the chamber 2. Similar technical features are referred to by the same reference-numerals as in figure 1a. During the operation of the position-sensitive detector 1 the micro-wire electrode 12 and the patterned foil electrode 13 are suspended inside the chamber 2 by the multi-connector means 24. The multi-connector means 24 are further comprised of male plug pins 57 which are attached to the vacuum chamber 2, and female socked pins 58 attached to each of the plurality of electrodes 10, each of the pins being electrically insulated from the others. The male plug pins 57 and the female socked pins 58 are made of a conductor such as copper, copper beryllium, stainless steal, aluminum, or titanium. The male plug pins 57 are further electronically connected to the transmission lines 20. Furthermore the male plug pins 57 and the female socked pins 58 can easily be disconnected by mechanically pulling them apart. The design of the multi-connector means 24 are such that this connection and disconnection can be repeated many times reliably. This allows the plurality of electrodes 10 to be easily removed from the chamber 2 and exchanged.

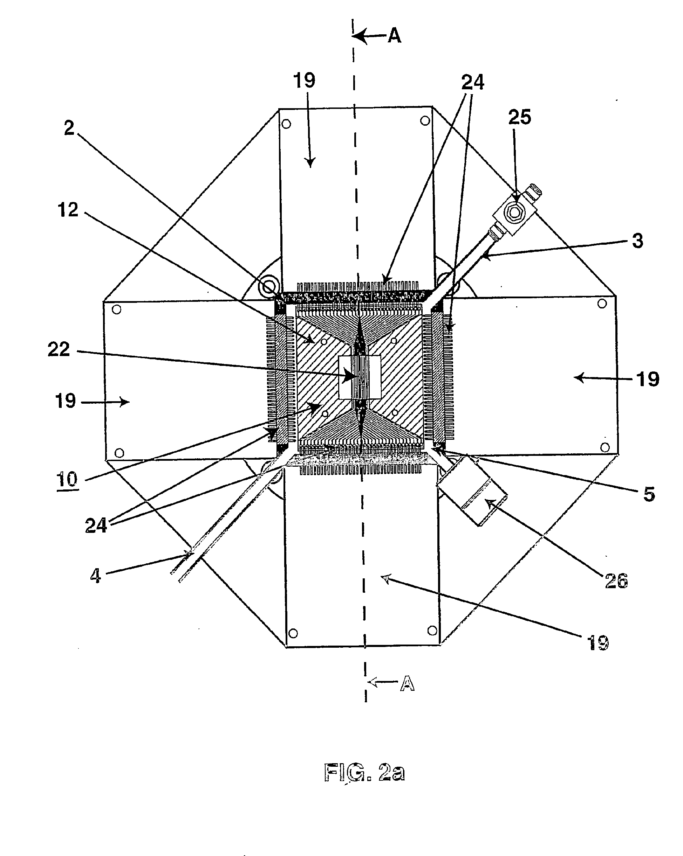

Figure 2a displays a cross-section of another preferred embodiment. Identical technical features are referred to by the same reference-numerals as in figure 1a. Within chamber 2 a plurality of electrodes 10 "stacked" upon each other are arranged. The direction of the beam is perpendicular to the surface of the drawing. Clearly depicted is a micro-wire electrode 12. The micro-wires 22 are connected to a metallic pattern 23. The metallic pattern 23 is itself connected to readout circuits 19, the multi-connector means 24 being integrated into said chamber 2. Further depicted are the gas inlet 3 that comprises a valve 25. Positioned opposite to the gas inlet 3 is the gas outlet 4. The chamber 2 further comprises a pressure gauge 26 to measure the pressure of the gas 5 and to produce electrical signals that may be used to control the valve 25 and/or a vacuum pump (not shown) to maintain a constant pressure within the chamber 2.

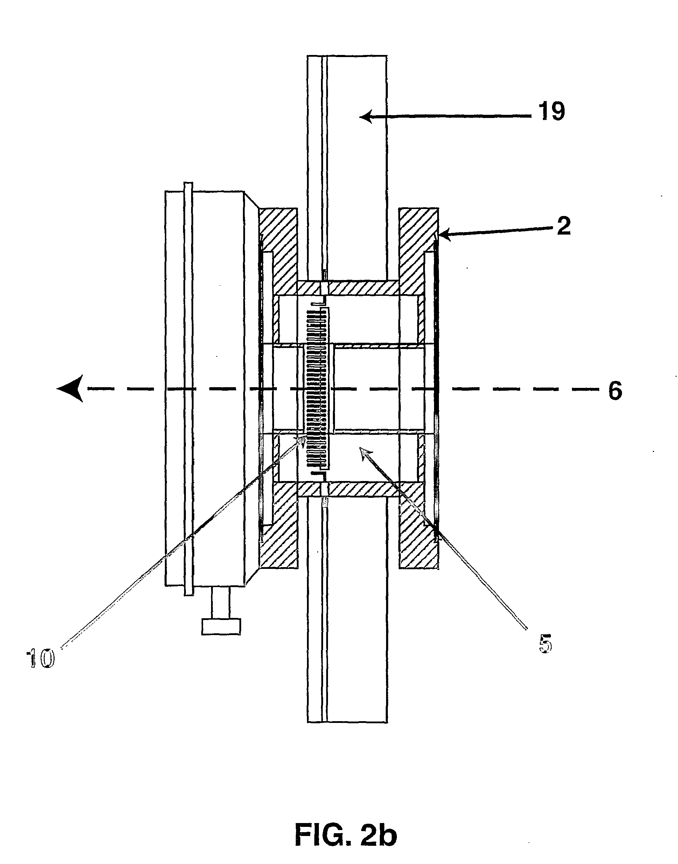

Figure 2b shows a cross-section of the same embodiment as displayed in Figure 2a along the phantom line A-A of figure 2a. Depicted are the readout circuits 19 positioned directly adjacent to the chamber 2, that may be filled with the gas 5. The beam 6 crosses the plurality of electrodes 10 under a 90° angle.

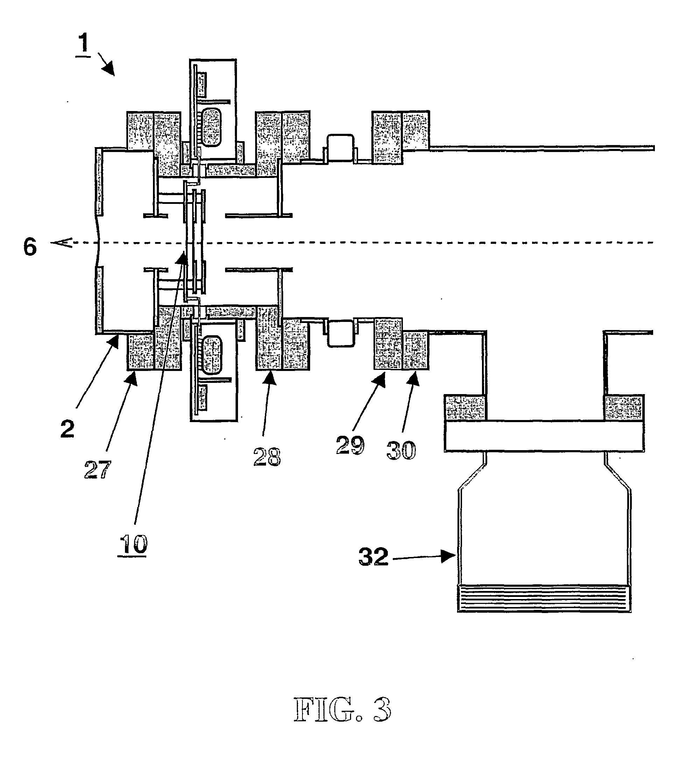

In figure 3 a different embodiment is displayed. Again technical features identical to those displayed in figures 1a, 1b, 2a, and 2b are referred to by the same reference-numerals. The chamber 2 of the position-sensitive detector 1 comprises different sections 27, 28, 29, 30, that are connected by detachable vacuum flanges to each other. Inside electrode section 28 the plurality of electrodes 10 is mounted. Again the beam 6 crosses the plurality of electrodes 10 under a right angle. Insulating section 29 contains an insulating tube 31 electrically insulating the electrode section 28 from beam entrance section 30. This way electromagnetic interference, which might be conducted and/or transmitted along the vacuum tubes enclosing the beam 6 on its path from the source are stopped from being transmitted to the electrode section 28, thereby reducing interference effects drastically and increasing the sensitivity of the position-sensitive detector 1. The beam entrance section 30 contains the gas outlet 4 to which a vacuum pump 32 is connected. The vacuum pump 32 used may be of turbo-molecular, ion-getter, sublimation, cryogenic, scroll, or rotary oil type. The entrance opening 8 and/or the exit opening 9 could be closed by a transparent window. Further they can be connected to a differential pumping section, thereby allowing a difference in gas pressure of several orders of magnitude between the chamber 2 and a connecting vacuum system attached to the position-sensitive detector 1. In a most preferred embodiment the position-sensitive detector 1 comprises disk valves attached to the entrance opening 8 and/or the exit opening 9. The disks may contain window inserts. An embodiment of the position-sensitive detector 1 comprising these disk valves with windows in the disks does not have to be disconnected from a vacuum system, when switching from a "gas-filled"-operating-mode to an "evacuated"-operating-mode. It is sufficient to just open/close one or both of the disk valves.

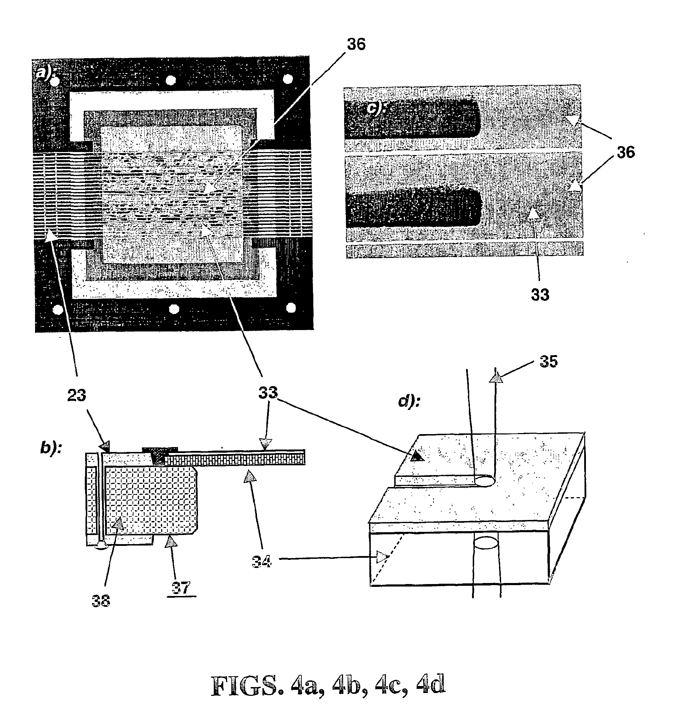

Further details of the structure and the manufacturing method for foil electrodes will be given with the reference to figures 4a, 4b, 4c and 4d. A metal layer typically made of gold, aluminum, or silver with a thickness between 10 nanometers and 10 microns is deposited onto a plastic foil 34 with a thickness between 0.1 and 100 microns, and a width and length between 1 centimeter and a few meters. Plastic foils 34 made of polyimide are especially suited for the use in high radiation and/or high-temperature environments. Electrode patterns with a resolution down to 1 micron are formed on the metal layer 33 by irradiating it with a laser beam 35 and scarming if across the surface to cut a desired pattern of segments 36. The crucial features of the laser beam 35 is that it vaporizes and cuts the metal layer 33, while leaving the plastic foil 34 undamaged, since the plastic foil 34 is transparent. The pattern, and thus the segments 36, may be of arbitrary geometrical shape. For example, the patterned foil electrode 12 being configured with 100 vertical (horizontal) strips and 100-micron spacing between each strip can measure the horizontal (vertical) projection of the radiation or particle beam 6, over an active area of 10 mm and at a resolution of 100 microns. The patterned circuit board 37 may be produced by any method known in the art.

The plastic foil 34 is mounted onto a patterned circuit board. The patterned circuit board 37 comprises an insulating frame 38. The insulating frame 38 is made of an insulator such as ceramic, sapphire, glass, quartz or glass epoxy. The patterned circuit board 37 further comprises the metallic pattern 23 made of metal such as copper, aluminum, gold, or silver. The segments 36 of the patterned metal layer 33 are aligned with the metallic pattern 23. To establish a mechanical and electric connection between the segments 36 and the metallic pattern 23 conductive paste 39 is printed on top of both in the vicinity of the interface. This way a very reliable electric connection is established. The conductive paste may be composed of silver-carbon. This type of connection is very well suited to be used in ultrahigh vacuum conditions, since it is not outgasing. Any other method to apply the conductive paste can be used as well.

One other method to produce patterned foil electrodes is to print the segments composed of metal directly onto the plastic foil.

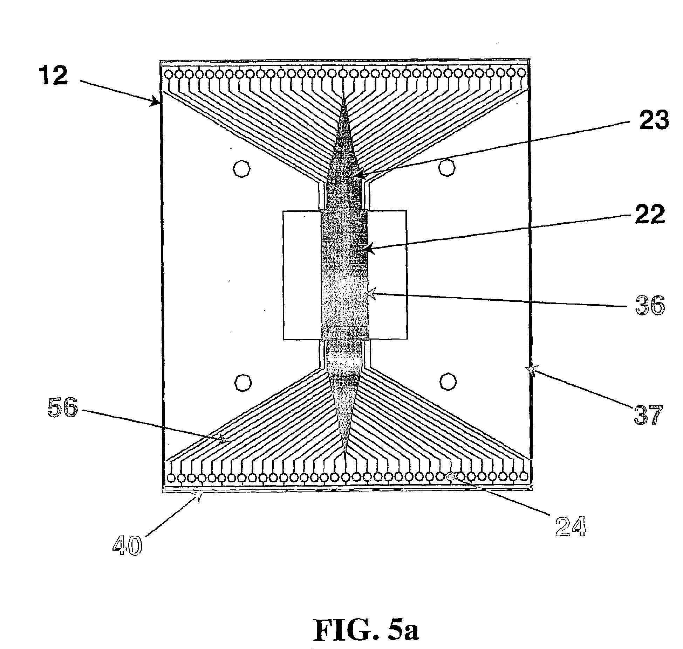

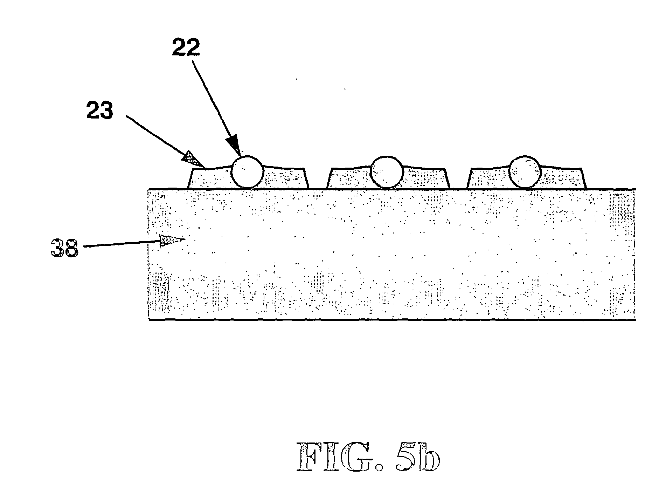

Figures 5a and 5b show a preferred embodiment for the micro-wire electrode 12. The segments 36 of said micro-wire electrode 12 is formed by micro-wires 22. These are connected to the metallic pattern 23, which is deposited on the insulating frame 38. The micro-wires 22 are made of conductors such as tungsten, gold-coated tungsten, gold, silver, carbon, silicon, silicon carbide, stainless steel, aluminum, or copper-beryllium, with diameters between 1 micron and 1 mm. The micro-wires 22 are arranged in a grid configuration with spacings down to 100 microns between each of them and bonded to the patterned circuit board 37. The bonding is done, for example, by stretching the micro-wires 22 on top of the metallic pattern 23, placing an electrode tip (not shown) made of a metal, such as tungsten, on top of them and applying a pulsed electric current. The resulting discharge fuses the micro-wires 22 into the metallic pattern 23, as shown in figure 5b. The micro-wires 22 mounted horizontally and vertically provide a vertical and horizontal projection, respectably, of the radiation or particle beam 6 to be measured. The metallic pattern 23 comprises circuit paths 56 that fan out towards an edge 40 of said micro-wire electrode 12. This makes it easier to connect a transmission line to each circuit path 56 being electrically connected to one segment 36 of the micro-wire electrode 12.

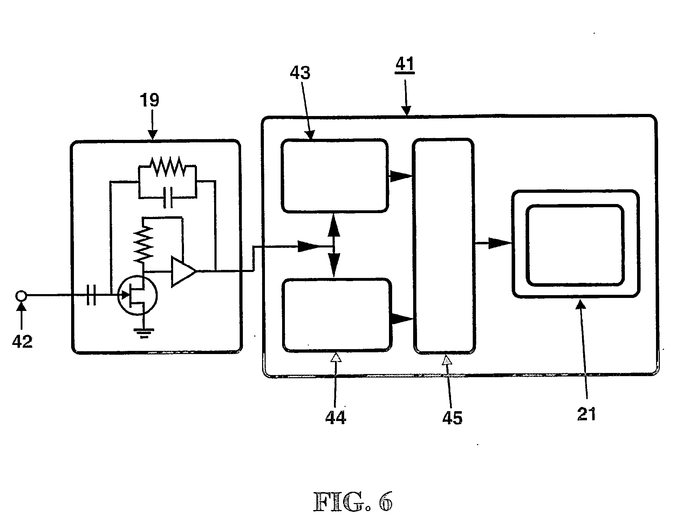

Figure 6 displays a schematic drawing of said readout circuits 19 in greater detail and of means for evaluating electronic signals from said readout circuits 19 being connected to said means 41, which electronically deduce at least one parameter characterizing said beam, to be measured. The readout circuits 19 receive the electronic signals from said elements of said plurality of patterned electrodes via an input 42. The readout circuits 19 comprise electronic amplifiers. The amplified signal is than transferred to the means 41 for evaluating those signals. The means 41 for evaluating the signals received from said readout circuits 19 comprise an integrator 43, an waveform recorder 44, an electronic interface 45 and display device 21. The intensity of the amplified signal is measured by integrator 43 while the timing profile is measured by waveform recorder 44. The data is transferred via the electronic interface 45 to the display device 21, where an image of the spatial profile, the intensity or dose, and for the time profile of the radiation or particle beam 6 is projected. Further the result of the evaluation can be supplied in electronic form.

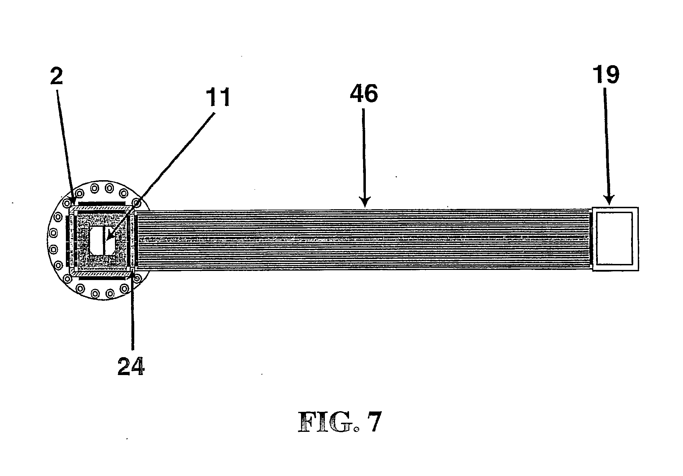

An example for a preferred embodiment of the readout circuits 19 will be described with the reference to figure 7. The chamber 2 containing said plurality of patterned electrodes 11 can be placed in extreme and hostile environments, such as pressures up to 10 Bars, magnetic fields between 0 and 5 Tesla and temperatures between -273 and 200 degrees Celsius. The more delicate readout circuits 19 are placed in a moderate environment at a distance of 0.1 to 100 meters away from the chamber 2, and connected to said plurality of patterned electrodes 11 using remote transmission lines 46. These may consist of a number of flexible or rigid cables of coaxial or twisted-pair design, having any diameter between 10 micron and 2 mm. They are normally made of a conductor such as tungsten, copper, stainless steel or aluminum, which is able to tolerate the harsh environments described above. Multiple pin connector 24 or other types of connectors may be formed as vacuum feedthrough that may be aligned along the walls of said chamber 2, to which the remote transmission lines are connected. The multiple pin connector 24 is connected to the segments of the plurality of patterned electrodes 11 by internal transmission lines.

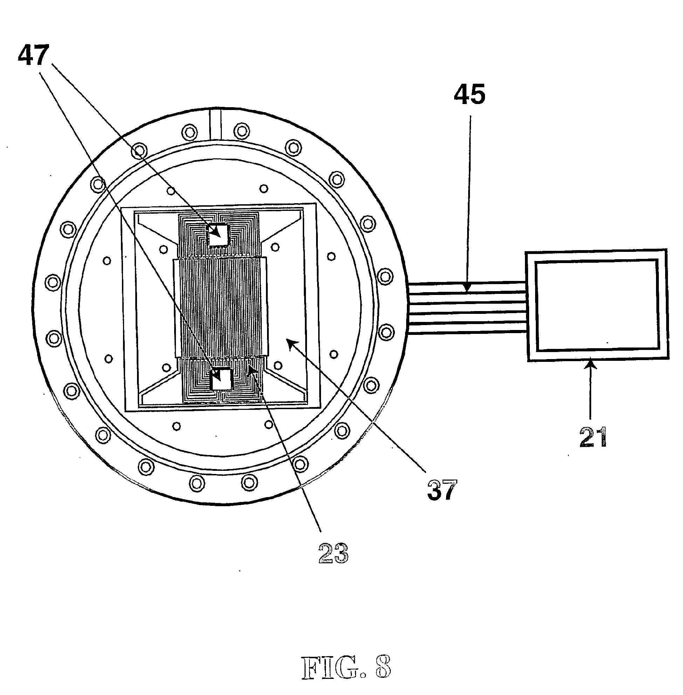

Another preferred embodiment of readout circuits 19 is described with reference to figure 8, wherein the readout circuits are miniaturized and encapsulated in an integrated circuit (IC) 47 with widths and lengths between 0.01 mm and 20 mm. Integrated circuit 47 is either mounted directly on the patterned circuit board 37 or near by with short internal transmission lines connecting it to the metallic pattern 23 of the patterned electrode. Data acquired by the integrated circuit 47 are transferred via the electronic interface 45 to the display device 21, such that the image of the spatial profile, intensity or dose, and timing profile of the radiation or particle beam is displayed.

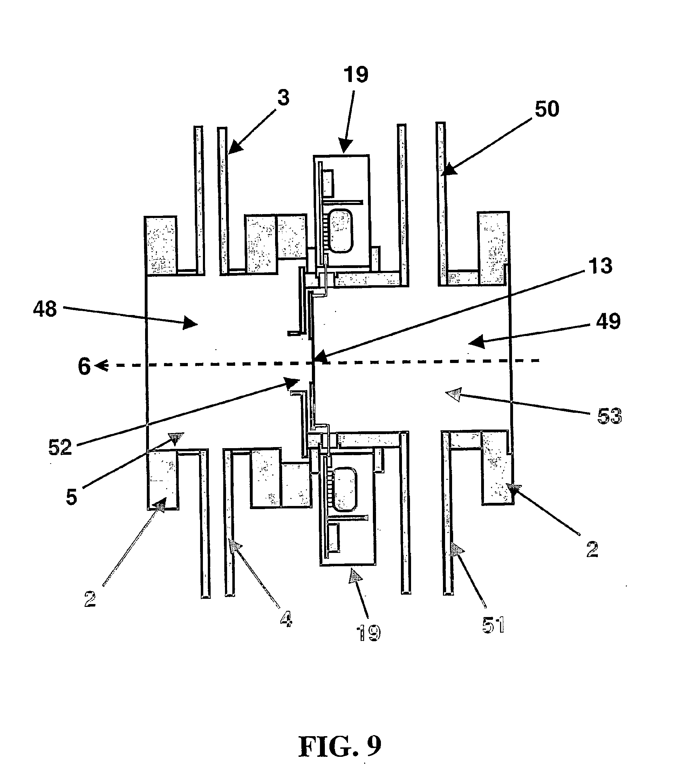

Figure 9 shows another preferred embodiment. It shows the position-sensitive detector 1 for measuring characteristics of a beam 6 of particles or radiation comprising: a chamber having a gas inlet 3 and a gas outlet 4; and a foil electrode 13 arranged inside said chamber 2; characterized in that, said foil electrode 13 comprises a pattern of segments 36 electrically insulated with respect to each other, and each of said segments 36 being separately electronically connectable, wherein said foil electrode 13 is mounted inside said chamber in such a way as to separate said chamber 2 into a portion 48 and another portion 49, sealing said portion 48 against said other portion 49 with respect to an exchange of a gas 5 between them, said portion 48 comprising said gas inlet 3 and said gas outlet 4, and said other portion 49 comprising another gas inlet 50 and another gas outlet 51. An aperture 52 having a typical diameter of 1 mm to 500 mm is hermetically sealed by the foil electrode 13 such that the flow of gas between said portion 48 and said other portion 49 of said chamber 2 is stopped. Using said gas inlet 3 and said other gas inlet 50, and said gas outlet 4 and said other gas outlet 51, said portion 48 and said other portion 49 may be filled with said gas 5 and another gas 53 having different pressures and compositions, or said portion 48 or said other portion 49 of said chamber 2 can be evacuated to vacuum. The foil electrode 13 now functions as a window that physically inhibits a gas exchange between said portion 48 and said other portion 49, as well as a detector that measures the spatial profile, intensity, and time structure of the radiation or particle beam 6 passing between said other portion 49 and said portion 48 of said chamber 2. This further increases the versatility of the detector.

The features disclosed the forgoing description, in the claims and/or in the accompanying drawings may, both separately and in any combination thereof, be material for realizing the invention in divers forms thereof.

## Claims

1. Position-sensitive detector (1) for measuring characteristics of a beam (6) of particles or radiation comprising:
- a chamber (2); and
- a plurality of electrodes (10) arranged in a parallel configuration inside said chamber (2), said plurality of electrodes (10) comprises a plurality of patterned electrodes (11), wherein each of said plurality of patterned electrodes (11) comprises a pattern of segments (36) electrically insulated with respect to each other, and each of said segments (36) being separately electronically connectable;
**characterized in that** said chamber (2) comprises multi-connector means (24) for individually detachably connecting each of said plurality of patterned electrodes (11) electrically to said multi-connector means (24) inside said chamber (2).

2. Detector according to claim 1, **characterized in that** said multi-connector means (24) are vacuum feedthroughs.

3. Detector according to any one of the preceding claims, **characterized in that** said plurality of patterned electrodes (11) is suspended by said multi-connector means (24).

4. Detector according to any one of the claims 1 to 3, **characterized in that** said plurality of electrodes (10) comprises foil electrodes and/or micro-wire electrodes only.

5. Detector according to any one of the preceding claims, **characterized in that** each of said segments (36) of said plurality of patterned electrodes (11) is connected to a separate readout circuit (19).

6. Detector according to claim 5, **characterized by** means (41) for evaluating electronic signals from said readout circuits (19) connected to said means (41), which electronically deduce at least one parameter characterizing said beam (6).

7. Detector according to any one of the preceding claims, **characterized in that** said chamber (2) comprises a gas inlet (3) for inserting gas (5) into said chamber and a gas outlet (4) for evacuating said chamber (2).

8. Detector according to any one of the claims 4 to 7, **characterized in that** said patterned foil electrode (13) separates said chamber (2) into a portion (48) and another portion (49), being substantially hermetically sealed with respect to fluid-exchange between each other.

9. Detector according to claim 8, **characterized in that** said portion (48) of said chamber (2) comprises said gas inlet (3) and said outlet (4), and said other portion comprises another gas inlet (50) and another gas outlet (51), such that said two portions can be filled with fluids and/or be evacuated separately.

10. Detector according to any one of the preceding claims, **characterized by** a voltage supply (16) to bias at least one of said plurality of electrodes (10).

11. Detector according to claim 10, **characterized in that** said voltage supply (16) is connected to said plurality of electrodes (10) to bias at least said one of said plurality of patterned electrodes (11) with a low voltage of a first sign, such that charged particles of an opposite sign impinging on said plurality of electrodes are absorbed and induce a signal, thus being capable to function as a Faraday cup detector.

12. Detector according to claim 10, **characterized in that** said voltage supply (16) is connected to said plurality of electrodes (10) and said chamber (2) is filled with said gas (5) such that an electric field (18) established between said plurality of electrodes (10) does not exceed a preferred upper limit of 100 V/mm in order to function as ionization chamber.

13. Detector according to claim 10, **characterized in that** said chamber (2) is filled with said gas (5) and said voltage supply (16) is connected to said plurality of electrodes (10) such that cylindrical electric fields exceeding 100 V/mm are established in the vicinity of wires of said micro-wire electrode (12) in order to function as multiple wire proportional chamber.

14. Detector according to claim 10, **characterized in that** said chamber (2) is filled with said gas (5) and said voltage supply (16) is connected to said plurality of electrodes (10) such that an electric field exceeding 100 V/mm is established in the vicinity of said foil electrode (13) in order to function as parallel plate avalanche chamber.

15. Detector according to claim 10, **characterized in that** said chamber (2) is evacuated and said voltage supply (16) is connected to said plurality of electrodes (10) such that a large negative voltage is applied to at least one of said plurality of patterned electrodes (10), in order to measure the current created in the segments (36) of said at least one of said plurality of patterned electrodes (11) by emitted secondary electrons in order to function as secondary electron emission chamber.

16. Method for manufacturing a position-sensitive detector (1) comprising a plurality of electrodes (10), wherein the method comprises the steps of:
- supplying a chamber (2);
- producing a plurality of patterned electrodes (11) being comprised in said plurality of electrodes (10), wherein the process of producing said plurality of patterned electrodes (11) comprises the steps of:
- supplying an insulating substrate frame (38);
- depositing a metallic pattern (23) on said insulating substrate frame (38); and
- attaching segments (36) to said metallic pattern (23) to define a pattern of said patterned electrode; and
- arranging said plurality of patterned electrodes (11) in a parallel configuration inside said chamber (2);
**characterized by** providing multi-connector means (24) and individually detachably connecting each of said plurality of patterned electrodes (11) electrically to said multi-connector means (24).

17. Method for producing a patterned electrode, the method comprising the steps of:
- supplying an insulating substrate frame (38);
- depositing a metallic pattern (23) on said insulating substrate frame (38);
- attaching segments (36) to said metallic pattern (23) to define a pattern of said patterned electrode; and
- configuring said patterned electrode so that said patterned electrode can be individually detachably electrically connected to multi-connector means (24).

18. Method according to claim 16 or 17, wherein said step of attaching said segments (36) to the metallic pattern (23) comprises the steps of:
- aligning said segments (36) on said metallic pattern (23);
- applying a conductive paste, in particular a conductive paste (39) composed of silver-carbon, on top of said metallic pattern (23) and said segments (26) at an interface between them; and
- electrically connecting said segments (23) and said metallic pattern (23) by said applied conductive paste.

19. Method according to claim 18, wherein said step of attaching said segments (36) to the metallic pattern (23) further comprises a step of curing of said conductive paste (39).

20. Method according to any one of the claims 16 to 19, wherein said step of depositing said metallic pattern (23) on said insulating substrate (38) comprises a step of printing of said metallic pattern (23).

21. Method according to any one of the claims 16 to 20, further comprising the steps of:
- supplying a plastic foil (34) being transparent to laser light;
- applying a metal layer (33) to said foil; and
- scanning laser light across said metal layer (33) to vaporize parts of said metal layer (33) thereby patterning said metal layer (33) into said segments (36) without destroying said plastic foil (34).

22. Method according to claim 16 or 17, wherein said step of attaching said segments (36) to the metallic pattern (23) comprises the steps of:
- stretching a micro-wire (22) on top of said metallic pattern (23);
- placing an electrode tip on top of said micro-wire (22) and said metallic pattern (23); and
- applying an pulsed electric current on said electrode tip to achieve a discharge to fuse said micro-wire (22) to said metallic pattern (23).

23. Method according to any one of the preceding claims 16 to 22, wherein an integrated circuit chip (47) comprising readout circuits (19) is connected to said metallic pattern (23), said integrated circuit chip (47) being capable to process signals from said segments (36).

24. Patterned electrode comprising:
- an insulating frame (38);
- a metallic pattern (23) deposited on said frame (38); and
- segments (36) connected to said metallic pattern (23), wherein said patterned electrode is configured to be individually detachably electrically connected to multi-connector means (24).

25. Patterned electrode according to claim 24, wherein the segments (36) are pads of a metal layer (33) being deposited on a plastic foil (34).

26. Patterned electrode according to claim 25, wherein the segments (36) are attached by conductive paste (39).

27. Patterned electrode according to claim 24, wherein the segments (36) are wires (22).

28. Patterned electrode according to claim 27, wherein the segments (36) are attached by discharge fusing.

29. Patterned electrode according to claim 27 or 28, wherein the micro-wires (22) are conductors selected out of the group comprising tungsten, gold, silver, carbon, silicon, silicon carbide, stainless steel, aluminum, and copper-beryllium.

30. Patterned electrode according to any one of the claims 24 to 29, wherein the material of said frame (38) is selected out of the group of material comprising ceramic, sapphire, glass, quartz, or glass epoxy.

31. Patterned electrode according to any one of the claims 24 to 30, **characterized in that** an integrated circuit (47) comprising a readout circuit (19) for each of said segments is connected directly to the metallic pattern (23) on said frame (38).

32. Detector according to any one of the claims 1 to 15, **characterized by** comprising an patterned electrode according to any one of the claims 24 to 31.

## Patentansprüche

1. Ortsempfindlicher Detektor (1) zum Messen von Eigenschaften eines Strahls (6) von Teilchen oder Strahlung, mit:
- einer Kammer (2) und
- mehreren Elektroden (10), die in einer parallelen Ausrichtung innerhalb der Kammer (2) angeordnet sind, wobei die mehreren Elektroden (10) mit mehreren gemusterten Elektroden (11) gebildet sind, wobei jede der mehreren gemusterten Elektroden (11) mit einem Muster von Abschnitten (36) gebildet ist, die jeweils elektrisch isoliert gegeneinander sind und wobei jeder der Abschnitte (36) einzeln elektrisch anschließbar ist,
**dadurch gekennzeichnet, dass** die Kammer (2) eine Mehrfachanschlusseinrichtung (24) aufweist, mit der jeder der mehreren gemusterten Elektroden (11) einzelnen lösbar elektrisch in der Kammer (2) verbunden ist.

2. Detektor nach Anspruch 1, **dadurch gekennzeichnet, dass** die Mehrfachanschlusseinrichtung eine Vakummdurchführung ist.

3. Detektor nach einem der vorangehenden Ansprüche, **dadurch gekennzeichnet, dass** die mehreren gemusterten Elektroden (11) von der Mehrfachanschlusseinrichtung (24) gehalten werden.

4. Detektor nach einem der Ansprüche 1 bis 3, **dadurch gekennzeichnet, dass** die mehreren Elektroden (10) nur Folienelektroden und / oder Mikrodrahtelektroden umfassen.

5. Detektor nach einem der vorangehenden Ansprüche, **dadurch gekennzeichnet, dass** jeder Abschnitt (36) der mehreren gemusterten Elektroden (11) mit einer separaten Ausleseschaltung (19) verbunden ist.

6. Detektor nach Anspruch 5, **gekennzeichnet durch** eine Einrichtung (41) zur Auswertung elektronischer Signale von den Ausleseschaltungen (19), die mit der Einrichtung verbunden sind, welche wenigstens einen Parameter elektronisch bestimmt, der den Strahl (6) kennzeichnet.

7. Detektor nach einem der vorangehenden Ansprüche, **dadurch gekennzeichnet, dass** die Kammer (2) mit einer Gaszuführung (3) zum Zuführen von Gas (5) in die Kammer (2) und einer Gasabführung zum Evakuieren der Kammer (2) gebildet ist.

8. Detektor nach einem der Ansprüche 4 bis 7, **dadurch gekennzeichnet, dass** die gemusterte Folienelektrode (13) die Kammer (2) in einen Bereich (48) und einen anderen Bereich (49) unterteilt, die bezüglich eines Austauschs eines Fluids untereinander hermetisch versiegelt sind.

9. Detektor nach Anspruch 8, dadurch **ge kennzeichnet**, dass der Bereich (48) der Kammer (2) mit der Gaszuführung (3) und der Gasabführung (4) gebildet ist und der andere Bereich (49) mit einer weiteren Gaszuführung (50) und einer weiteren Gasabführung (51) gebildet ist, derart, dass die beiden Bereiche separat mit einem Fluid gefüllt und / oder evakuiert werden können.

10. Detektor nach einem der vorangehenden Ansprüche, **gekennzeichnet durch** eine Spannungsquelle (16), um wenigstens eine der mehreren Elektroden (10) mit einer Vorspannung zu beaufschlagen.

11. Detektor nach Anspruch 10, **dadurch gekennzeichnet, dass** die Spannungsquelle (16) mit den mehreren Elektroden (10) verbunden ist, um wenigstens eine der gemusterten Elektroden (11) mit einer geringen Vorspannung mit einem ersten Vorzeichen zu beaufschlagen, derart, dass geladene Teilchen mit einem entgegen gesetzten Vorzeichen, die auf die mehreren Elektroden aufprallen, abgefangen werden und ein Signal auslösen, so dass der Detektor als Faradaybecherdetektor wirkt.

12. Detektor nach Anspruch 10, **dadurch gekennzeichnet, dass** die Spannungsquelle (16) mit den mehreren Elektroden (10) verbunden ist und die Kammer (2) mit dem Gas (5) gefüllt ist, derart, dass ein elektrisches Feld (18) zwischen den mehreren Elektroden (10) nicht eine bevorzugte obere Grenze von 100 V/mm überschreitet, so dass der Detektor als Ionisierungskammer wirkt.

13. Detektor nach Anspruch 10, **dadurch gekennzeichnet, dass** die Kammer (2) mit dem Gas (5) gefüllt ist und die Spannungsquelle (16) mit den mehreren Elektroden (10) verbunden ist, derart, dass zylinderförmige elektrische Felder, die größer als 100 V/mm sind, in der Umgebung von Drähten der Mikrodrahtelektrode (12) aufgebaut werden, so dass der Detektor als Mehrfachdrahtproportionalkammer wirkt.

14. Detektor nach Anspruch 10, **dadurch gekennzeichnet, dass** die Kammer (2) mit dem Gas (5) gefüllt ist und die Spannungsquelle (16) mit den mehreren Elektroden (10) verbunden ist, derart, dass ein elektrisches Feld, das größer als 100 V/mm ist, in der Umgebung der Folienelektrode (13) gebildet ist, so dass der Detektor als Parallelplattenlawinenkammer wirkt.

15. Detektor nach Anspruch 10, **dadurch gekennzeichnet, dass** die Kammer (2) evakuiert ist und die Spannungsquelle (16) mit den mehreren Elektroden (10) verbunden ist, derart, dass eine große negative Spannung an wenigstens eine der mehreren gemusterten Elektroden (11) angelegt ist, um den in den Abschnitten (36) der mehreren gemusterten Elektroden (11) erzeugten Strom mittels emittierter Sekundärelektronen zu messen, so dass der Detektor als Sekundäremissionselektronenkammer wirkt.

16. Verfahren zum Herstellen eines ortsempfindlichen Detektors (1) mit mehreren Elektroden (10), wobei das Verfahren die folgenden Schritte umfasst:
- Bereitstellen einer Kammer (2),
- Herstellen von mehreren gemusterten Elektroden (11), die von den mehreren Elektroden (10) umfasst sind, wobei der Schritt des Herstellens der mehreren gemusterten Elektroden (11) die folgenden Schritte umfasst:
- Bereitstellen eine isolierenden Substratrahmens (38),
- Abscheiden eines metallischen Musters (23) auf dem isolierenden Substratrahmen (38) und
- Befestigen von Abschnitten (36) an dem metallischen Muster (23), um ein Muster der gemusterten Elektrode zu bestimmen, und
- Anordnen der mehreren gemusterten Elektroden (11) in einer parallelen Ausrichtung innerhalb der Kammer (2),
**gekennzeichnet, durch** ein Bereitstellen einer Mehrfachanschlusseinrichtung (24) und ein einzelnes lösbares elektrisches Verbinden von jeder der mehreren gemusterten Elektroden (11) mit der Mehrfachanschlusseinrichtung (24).

17. Verfahren zum Herstellen einer gemusterten Elektrode, mit folgenden Schritten:
- Bereitstellen eines isolierenden Substratrahmens (38),
- Abscheiden eines metallischen Musters (23) auf dem isolierenden Substratrahmen (38),
- Befestigen von Abschnitten (36) an dem metallischen Muster (23), um ein Muster der gemusterten Elektrode (11) zu bestimmen, und
- Konfigurieren der gemusterten Elektrode (11), derart, dass die gemusterte Elektrode (11) einzeln lösbar elektrisch mit der Mehrfachanschlusseinrichtung (24) verbunden werden kann.

18. Verfahren nach Anspruch 16 oder 17, wobei der Schritt des Befestigens der Abschnitte (36) an dem metallischen Muster (23) die Schritte umfasst:
- Ausrichten der Abschnitte (36) auf dem metallischen Muster (23),
- Aufbringen eines leitfähigen Klebemittels (39), insbesondere ein leitfähiges Klebemittel, das aus Silberkohlenstoff besteht, auf einer Oberseite des metallischen Musters (23) und der Abschnitte (36) an einer Grenzfläche zwischen diesen, und
- Elektrisches Verbinden der Abschnitte (36) und des metallischen Muster (23) mittels des leitfähigen Klebemittels (39).

19. Verfahren nach Anspruch 18, wobei der Schritt des Befestigen der Abschnitte (36) an dem metallischen Muster (23) weiter ein Aushärten des leitfähigen Klebemittels (39) umfasst.

20. Verfahren nach einem der Ansprüche 16 bis 19, wobei der Schritt des Abscheidens des metallischen Musters (23) auf dem isolierenden Substratrahmen (38) einen Schritt des Druckens des metallischen Musters (23) umfasst.

21. Verfahren nach einem der Ansprüche 16 bis 20, weiter die folgenden Schritte umfassend:
- Bereitstellen einer Kunststofffolie (34), die für Laserlicht durchlässig ist,
- Aufbringen einer Metallschicht (33) auf die Folie und
- Abtasten der Metallschicht (33) mittels Laserlicht, um Teile der Metallschicht zu verdampfen, wodurch die Metallschicht (33) in die Abschnitte (36) gemustert wird, ohne dass die Kunststofffolie (34) zerstört wird.

22. Verfahren nach einem der Ansprüche 16 oder 17, wobei der Schritt des Befestigens der Abschnitte (36) an dem metallischen Muster (23) die Schritte umfasst:
- Spannen eines Mikrodrahts (22) auf der Oberseite des metallischen Musters (23),
- Anordnen einer Elektrodenspitze auf der Oberseite des Mikrodrahts (22) und des metallischen Musters (23) und
- Einbringen eines gepulsten elektrischen Stroms auf die Elektrodenspitze, um mittels einer Entladung den Mikrodraht (22) auf das metallische Muster (23) aufzuschmelzen.

23. Verfahren nach einem der vorangehenden Ansprüche 16 bis 22, wobei ein integrierter Schaltungschip (47) mit Ausleseschaltungen (19) mit dem metallischen Muster (23) verbunden wird, wobei der integrierte Schaltungschip (47) konfiguriert ist, Signale von den Abschnitten (36) zu verarbeiten.

24. Gemusterte Elektrode, mit
- einem isolierenden Rahmen (38),
- einem metallischen Muster (23), dass auf dem Rahmen (38) abgeschieden ist, und
- Abschnitten (36), die mit dem metallischen Muster (23) verbunden sind,
wobei die gemusterte Elektrode konfiguriert ist, einzeln lösbar elektrisch mit einer Mehrfachanschlusseinrichtung (24) verbunden zu werden.

25. Gemusterte Elektrode nach Anspruch 24, wobei die Abschnitte (36) Teile einer Metallschicht (33) sind, die auf einer Kunststofffolie (34) abgeschieden ist.

26. Gemusterte Elektrode nach Anspruch 25, wobei die die Abschnitte (36) mittels eines leitfähigen Klebemittels (39) befestigt sind.

27. Gemusterte Elektrode nach Anspruch 24, wobei die Abschnitte (36) Drähte (22) sind.

28. Gemusterte Elektrode nach Anspruch 27, wobei die Abschnitte (36) mittels einer Entladungsschmelze befestigt sind.

29. Gemusterte Elektrode nach einem der Ansprüche 27 oder 28, wobei die Mikrodrähte (22) Leiter ausgewählt aus der Gruppe Wolfram, Gold, Silber, Kohlenstoff, Silizium, Siliziumkarbid, Edelstahl, Aluminium und Kupferberyllium sind

30. Gemusterte Elektrode nach einem der Ansprüche 24 bis 29, wobei das Material des Rahmens (38) ausgewählt ist aus der Gruppe Keramik, Saphir, Glass, Quartz und Glassepoxid.

31. Gemusterte Elektrode nach einem der Ansprüche 24 bis 30, **dadurch gekennzeichnet, dass** ein integrierter Schaltungschip (47) mit einer Ausleseschaltung (19) für jeden der Abschnitte (36) direkt mit dem metallischen Muster (23) auf dem Rahmen (38) verbunden ist.

32. Detektor nach einem der Ansprüche 1 bis 15, **gekennzeichnet durch** eine gemusterte Elektrode (11) nach einem der Ansprüche 24 bis 31.

## Revendications

1. Détecteur sensible à la position (1) pour mesurer des caractéristiques d'un faisceau (6) de particules ou de rayonnement comprenant :
- une chambre (2) ; et
- une pluralité d'électrodes (10) agencées dans une configuration parallèle à l'intérieur de ladite chambre (2), ladite pluralité d'électrodes (10) comprend une pluralité d'électrodes à motif (11), dans lequel chacune de ladite pluralité d'électrodes à motif (11) comprend un motif de segments (36) isolés électriquement l'un de l'autre, et chacun desdits segments (36) peut être connecté électroniquement de manière distincte ;
**caractérisé en ce que** ladite chambre (2) comprend un moyen à plusieurs connecteurs (24) pour connecter individuellement de manière détachable chacune de ladite pluralité d'électrodes à motif (11) électriquement au dit moyen à plusieurs connecteurs (24) à l'intérieur de ladite chambre (2).

2. Détecteur selon la revendication 1, **caractérisé en ce que** ledit moyen à plusieurs connecteurs (24) est une connexion d'interface sous vide.

3. Détecteur selon l'une quelconque des revendications précédentes, **caractérisé en ce que** ladite pluralité d'électrodes à motif (11) est suspendue par ledit moyen à plusieurs connecteurs (24).

4. Détecteur selon l'une quelconque des revendications 1 à 3, **caractérisé en ce que** ladite pluralité d'électrodes (10) comprend des électrodes à feuilles et/ou des électrodes à microfils uniquement.

5. Détecteur selon l'une quelconque des revendications précédentes, **caractérisé en ce que** chacun desdits segments (36) de ladite pluralité d'électrodes à motif (11) est connecté à un circuit de lecture distinct (19).

6. Détecteur selon la revendication 5, **caractérisé par** un moyen (41) pour évaluer des signaux électroniques desdits circuits de lecture (19) connectés au dit moyen (41), qui déduit électroniquement au moins un paramètre caractérisant ledit faisceau (6).

7. Détecteur selon l'une quelconque des revendications précédentes, **caractérisé en ce que** ladite chambre (2) comprend une entrée de gaz (3) pour introduire un gaz (5) dans ladite chambre et une sortie de gaz (4) pour évacuer ladite chambre (2).

8. Détecteur selon l'une quelconque des revendications 4 à 7, **caractérisé en ce que** ladite électrode à feuille à motif (13) sépare ladite chambre (2) en une partie (48) et une autre partie (49), sensiblement hermétiques l'une par rapport à l'autre en ce qui concerne l'échange de fluide.

9. Détecteur selon la revendication 8, **caractérisé en ce que** ladite partie (48) de ladite chambre (2) comprend ladite entrée de gaz (3) et ladite sortie (4), et ladite autre partie comprend une autre entrée de gaz (50) et une autre sortie de gaz (51), de sorte que lesdites deux parties puissent être remplies de fluide et/ou évacuées séparément.

10. Détecteur selon l'une quelconque des revendications précédentes, **caractérisé par** une alimentation de tension (16) pour polariser au moins l'une de ladite pluralité d'électrodes (10).

11. Détecteur selon la revendication 10, **caractérisé en ce que** ladite alimentation de tension (16) est connectée à ladite pluralité d'électrodes (10) pour polariser ladite au moins une de ladite pluralité d'électrodes à motif (11) avec une basse tension d'un premier signe, de sorte que les particules chargées d'un signe opposé empiétant sur ladite pluralité d'électrodes soient absorbées et induisent un signal, en étant ainsi capable de fonctionner en tant que détecteur à chambre d'ionisation de Faraday.

12. Détecteur selon la revendication 10, **caractérisé en ce que** ladite alimentation de tension (16) est connectée à ladite pluralité d'électrodes (10) et ladite chambre (2) est remplie dudit gaz (5) de sorte qu'un champ électrique (18) établi entre ladite pluralité d'électrodes (10) ne dépasse pas une limite supérieure préférée de 100 V/mm pour fonctionner en tant que chambre d'ionisation.

13. Détecteur selon la revendication 10, **caractérisé en ce que** ladite chambre (2) est remplie dudit gaz (5) et ladite alimentation de tension (16) est connectée à ladite pluralité d'électrodes (10) de sorte que des champs électriques cylindriques dépassant 100 V/mm soient établis à proximité des fils de ladite électrode à microfils (12) afin de fonctionner en tant que chambre proportionnelle à fils multiples.

14. Détecteur selon la revendication 10, **caractérisé en ce que** ladite chambre (2) est remplie dudit gaz (5) et ladite alimentation de tension (16) est connectée à ladite pluralité d'électrodes (10) de sorte qu'un champ électrique dépassant 100 V/mm soit établi à proximité de ladite électrode à feuille (13) pour fonctionner en tant que chambre à plaques parallèles en avalanche.

15. Détecteur selon la revendication 10, **caractérisé en ce que** ladite chambre (2) est évacuée et ladite alimentation de tension (16) est connectée à ladite pluralité d'électrodes (10) de sorte qu'une grande tension négative soit appliquée à au moins une de ladite pluralité d'électrodes à motif (10), pour mesurer le courant créé dans les segments (36) de ladite au moins une de ladite pluralité d'électrodes à motif (11) par des électrons secondaires émis afin de fonctionner en tant que chambre d'émission d'électrons secondaire.

16. Procédé pour fabriquer un détecteur sensible à la position (1) comprenant une pluralité d'électrodes (10), dans lequel le procédé comprend les étapes de :
- fournir une chambre (2) ;
- produire une pluralité d'électrodes à motif (11) comprises dans ladite pluralité d'électrodes (10), dans lequel le processus de production de ladite pluralité d'électrodes à motif (11) comprend les étapes :
- fournir un cadre de substrat isolant (38) ;
- déposer un motif métallique (23) sur ledit cadre de substrat isolant (38) ; et
- attacher des segments (36) au dit motif métallique (23) pour définir un motif de ladite électrode à motif ; et
- agencer ladite pluralité d'électrodes à motif (11) dans une configuration parallèle à l'intérieur de ladite chambre (2) ;
**caractérisé par** les étapes consistant à fournir un moyen à plusieurs connecteurs (24) et à connecter individuellement de manière détachable chacune de ladite pluralité d'électrodes à motif (11) électriquement au dit moyen à plusieurs connecteurs (24).

17. Procédé pour produire une électrode à motif, le procédé comprenant les étapes de :
- fournir un cadre de substrat isolant (38) ;
- déposer un motif métallique (23) sur ledit cadre de substrat isolant (38) ;
- attacher des segments (36) au dit motif métallique (23) pour définir un motif de ladite électrode à motif ; et
- configurer ladite électrode à motif de sorte que ladite électrode à motif puisse être connectée électriquement et individuellement de manière détachable au moyen à plusieurs connecteurs (24).

18. Procédé selon la revendication 16 ou 17, dans lequel ladite étape consistant à attacher lesdits segments (36) au motif métallique (23) comprend les étapes de :
- aligner lesdits segments (36) sur ledit motif métallique (23) ;
- appliquer une pâte conductrice, en particulier une pâte conductrice (39) composée d'argent-carbone, dessus ledit motif métallique (23) et lesdits segments (26) à une interface entre eux ; et
- connecter électriquement lesdits segments (23) et ledit motif métallique (23) par ladite pâte conductrice appliquée.

19. Procédé selon la revendication 18, dans lequel ladite étape consistant à attacher lesdits segments (36) au motif métallique (23) comprend en outre une étape consistant à durcir ladite pâte conductrice (39).

20. Procédé selon l'une quelconque des revendications 16 à 19, dans lequel ladite étape consistant à déposer ledit motif métallique (23) sur ledit substrat isolant (38) comprend une étape consistant à imprimer ledit motif métallique (23).

21. Procédé selon l'une quelconque des revendications 16 à 20, comprenant en outre les étapes de :
- fournir une feuille de plastique (34) transparente à la lumière laser ;
- appliquer une couche de métal (33) sur ladite feuille ; et
- balayer une lumière laser sur ladite couche de métal (33) pour vaporiser des parties de ladite couche de métal (33) en créant de ce fait un motif sur ladite couche de métal (33) comportant lesdits segments (36) sans détruire ladite feuille de plastique (34).

22. Procédé selon la revendication 16 ou 17, dans lequel ladite étape consistant à attacher lesdits segments (36) au motif métallique (23) comprend les étapes de :
- étirer un microfil (22) dessus ledit motif métallique (23) ;
- placer un embout d'électrode dessus ledit microfil (22) et ledit motif métallique (23) ; et
- appliquer un courant électrique pulsé sur ledit embout d'électrode pour obtenir une décharge afin de faire fondre ledit microfil (22) sur ledit motif métallique (23).

23. Procédé selon l'une quelconque des revendications 16 à 22, dans lequel une puce à circuits intégrés (47) comprenant des circuits de lecture (19) est connectée au dit motif métallique (23), ladite puce à circuits intégrés (47) étant capable de traiter des signaux desdits segments (36).

24. Electrode à motif comprenant :
- un cadre isolant (38) ;
- un motif métallique (23) déposé sur ledit cadre (38) ; et
- des segments (36) connectés au dit motif métallique (23), dans lequel ladite électrode à motif est configurée pour être connectée électriquement et individuellement de manière détachable à un moyen à plusieurs connecteurs (24).

25. Electrode à motif selon la revendication 24, dans laquelle les segments (36) sont des tampons d'une couche de métal (33) étant déposée sur une feuille de plastique (34).

26. Electrode à motif selon la revendication 25, dans laquelle les segments (36) sont attachés par une pâte conductrice (39).

27. Electrode à motif selon la revendication 24, dans laquelle les segments (36) sont des fils (22).

28. Electrode à motif selon la revendication 27, dans laquelle les segments (36) sont attachés par fusion à décharge.

29. Electrode à motif selon la revendication 27 ou 28, dans laquelle les microfils (22) sont des conducteurs sélectionnés dans le groupe se composant de tungstène, d'or, d'argent, de carbone, de silicium, de carbure de silicium, d'acier inoxydable, d'aluminium et de cuivre-béryllium.

30. Electrode à motif selon l'une quelconque des revendications 24 à 29, dans laquelle le matériau dudit cadre (38) est sélectionné dans le groupe de matériaux se composant de céramique, de saphir, de verre, de quartz ou d'époxyde de verre.

31. Electrode à motif selon l'une quelconque des revendications 24 à 30, **caractérisée en ce qu'**un circuit intégré (47) comprenant un circuit de lecture (19) pour chacun desdits segments est connecté directement au motif métallique (23) sur ledit cadre (38).

32. Détecteur selon l'une quelconque des revendications 1 à 15, **caractérisé en ce qu'**il comprend une électrode à motif selon l'une quelconque des revendications 24 à 31.
